# FASCICULE DE BREVET EUROPEEN

(11) **EP 3 224 243 B1**
(45) Date de publication et mention de la délivrance du brevet: **09.03.2022**
(21) Numéro de dépôt: 15808731.2
(22) Date de dépôt: 25.11.2015
(51) Int. Cl.: C07C 333/16

(54) **PROCÉDÉ DE PRÉPARATION DE DITHIOCARBAMATES DE ZINC**
VERFAHREN ZUR HERSTELLUNG VON ZINKDITHIOCARBAMATEN
METHOD FOR PRODUCING ZINC DITHIOCARBAMATES

(30) Priorité: 25.11.2014 FR 1461427
(43) Date de publication de la demande: 04.10.2017
(73) Titulaire: MLPC International, 40370 Rion-Des-Landes (FR)
(72) Inventeur: AUBERT, Thierry, 64230 Lescar (FR)
(74) Mandataire: Arkema Patent
(86) Numéro de dépôt international: PCT/FR2015/053205
(87) Numéro de publication internationale: WO 2016/083733

(56) Documents cités:
- EP-A1- 0 288 819
- EP-A2- 0 727 429
- EP-A2- 1 142 869
- WO-A1-00/48463
- US-A- 2 406 960
- ONWUDIWE DAMIAN C ET AL: "Synthesis, spectroscopic characterization and behavior of AC impedance spectroscopy of Cd(II) bis(N-para-methylphenyl dithiocarbamate)", ELECTROCHIMICA ACTA, vol. 104, 25 avril 2013 (2013-04-25), pages 19-25, XP028594207, ISSN: 0013-4686, DOI: 10.1016/J.ELECTACTA.2013.04.081

## Description

L'invention concerne le procédé de fabrication d'accélérateurs de vulcanisation de type dithiocarbamates de zinc, agents bien connus et utilisés couramment pour la vulcanisation de divers types de caoutchouc.

Les dithiocarbamates de zinc sont très utilisés dans l'industrie du caoutchouc, du fait de leurs excellentes performances, que ce soit du point de vue de la cinétique de vulcanisation ou des propriétés physiques des caoutchoucs vulcanisés au moyen desdits dithiocarbamates.

Cependant, l'un des problèmes auquel doit faire face l'industrie du caoutchouc est la formation de nitrosamines cancérigènes volatiles au cours de la vulcanisation lors de l'utilisation de la plupart des dithiocarbamates de zinc classiques tels que les diméthyl-, diéthyl- ou dibutyl-dithiocarbamates de zinc.

Afin de pallier ce problème, l'une des solutions les plus utilisées actuellement est de remplacer les dithiocarbamates mentionnés ci-dessus par le dibenzyl-dithiocarbamate de zinc, également identifié par son acronyme ZBEC (ou ZBDC ou ZTC). En effet, le ZBEC génère une nitrosamine non volatile à laquelle les travailleurs ne sont donc pas exposés, d'où la dénomination anglo-saxonne de « nitrosamine-safe dithiocarbamate » pour cet accélérateur de vulcanisation.

Ces accélérateurs de vulcanisation, sous forme de poudre, doivent être incorporés dans le caoutchouc solide, par exemple à l'aide d'un mélangeur à cylindres, afin que la dispersion de cette poudre soit la plus homogène possible dans la matrice caoutchouc, ceci pour assurer une vulcanisation homogène du caoutchouc et des propriétés physico-chimiques optimales.

Plus les poudres à disperser ont une granulométrie régulière et une taille de particules petite, meilleure est la dispersion. Par ailleurs, plus la granulométrie du produit est petite, plus le procédé de fabrication du produit est délicat et ceci pour deux raisons principales : au cours de la cristallisation (ou précipitation) du produit, généralement en phase aqueuse, la formation de très petites particules génère des problèmes d'agitation pouvant aller jusqu'à la formation d'un gel impossible à agiter ; d'autre part, les étapes de finition, à savoir filtration et séchage du produit sont d'autant plus délicates que la taille des particules est petite. Il peut s'avérer en particulier très long et très difficile (besoin de fortes pressions) de filtrer un produit dont la granulométrie médiane est de l'ordre du micromètre en volume.

Il y a donc un compromis à trouver concernant la taille des particules du produit pour satisfaire ces deux objectifs contradictoires : pouvoir disposer d'un procédé de fabrication industriel techniquement et économiquement viable et disperser au mieux le produit fini dans le caoutchouc avant d'effectuer la vulcanisation. Pour la plupart des accélérateurs de vulcanisation, ce compromis se situe généralement pour une granulométrie médiane en volume Dv50 (ou x₅₀) comprise entre 10 µm et 60 µm, mieux encore entre 15 µm et 40 µm, celle-ci étant mesurée avec les techniques bien connues de l'homme du métier, telles que par exemple avec un granulomètre laser. La définition de la granulométrie médiane en volume Dv50 ainsi que sa méthode de mesure sont décrites par exemple dans la norme ISO 13320 : 2009(E).

Pour les dithiocarbamates de zinc en particulier, il est souvent difficile d'atteindre de telles valeurs de taille de particules car les dithiocarbamates de zinc cristallisent généralement sous la forme de très petites particules, typiquement avec une valeur médiane de taille des particules largement inférieures à 10 µm et plus généralement entre 2 µm et 5 µm en volume, tout particulièrement dans le cas du ZBEC.

Par une optimisation des conditions du procédé, il est souvent possible d'atteindre des tailles de particules de l'ordre de 15 µm, mais même dans ce cas, les étapes de finition du procédé telles que la filtration et le séchage sont longues et la productivité globale du procédé est très limitée ; de plus, dans le cadre de la production à grande échelle de quantités importantes de produits se pose la question importante de la nature et de la quantité de rejets ; en particulier, beaucoup de procédés proposés dans la littérature utilisent le chlorure de zinc comme source de zinc; or, les rejets chlorurés, qui représentent la très grande majorité des effluents salins dans l'environnement, sont à limiter au maximum.

Ainsi, plusieurs demandes récentes, entre autres les demandes CN101955452, CN101081828 et CN1827622, décrivent des procédés de fabrication de dithiocarbamates de zinc tels que le ZBEC à partir de chlorure de zinc (ZnCl₂) comme source de zinc. Cette source de zinc permet une réaction rapide et complète avec un dithiocarbamate soluble dans l'eau (tel qu'un dithiocarbamate de sodium ou de potassium) pour conduire à un dithiocarbamate de zinc solide en suspension dans l'eau. De tels procédés de synthèses sont à proscrire et donc inutilisables pour produire des quantités importantes de produits, en raison de la coproduction d'effluents salins chlorurés non désirables, après filtration du dithiocarbamate de zinc obtenu en fin de réaction.

D'autres procédés décrits dans la littérature utilisent le sulfate de zinc pour la préparation de dithiocarbamates de zinc, toujours à partir d'un dithiocarbamate soluble dans l'eau ; on peut citer par exemple US2406960 pour le ZBEC.

Tous les procédés utilisant la réaction entre un sel de zinc soluble, tel que le chlorure ou le sulfate de zinc, et un dithiocarbamate soluble, tel qu'un dithiocarbamate de sodium ou de potassium, conduisent à une réaction rapide entre les deux réactifs avec une formation de très nombreux cristaux de dithiocarbamates de zinc insolubles dont la taille est généralement comprise entre 2 µm et 30 µm, le plus souvent entre 5 µm et 20 µm.

D'autres procédés décrits dans l'art antérieur utilisent l'oxyde de zinc comme source de zinc. Cependant, l'oxyde de zinc n'est pas soluble dans le milieu réactionnel, et est donc beaucoup moins réactif que le chlorure ou le sulfate de zinc précités ; il en résulte des procédés conduisant à des tailles de cristaux de dithiocarbamates de zinc beaucoup plus importantes. Dans ce cas, la productivité du procédé est améliorée car les problèmes d'agitation sont moindres, ce qui permet de concentrer le milieu réactionnel, et les étapes de filtration et de séchage sont plus rapides ; néanmoins, ces procédés sont généralement moins efficaces, du fait de la faible réactivité de l'oxyde de zinc, et conduisent à des rendements faibles, donc des rejets organiques beaucoup plus importants.

Parmi les procédés mettant en œuvre l'oxyde de zinc comme source de zinc, on peut citer par exemple ceux décrits dans DE4102337 et CN103755613 qui nécessitent l'emploi d'un solvant aromatique. Ce procédé est donc non seulement coûteux, en raison de la nécessité de recycler le solvant, mais aussi présente des risques d'hygiène, de sécurité et d'environnement, en raison de la toxicité avérée des solvants aromatiques.

Comme autre procédé mettant en œuvre l'oxyde de zinc comme source de zinc, on peut citer celui décrit dans CN102276509 qui n'utilise pas de solvants comme les procédés décrits dans DE4102337 et CN103755613 et qui conduisent à des produits de granulométrie médiane élevée. Le document CN102276509 annonce des rendements supérieurs à 99% avec des puretés supérieures ou égales à 97%, cependant il s'est avéré difficile de reproduire des rendements supérieurs à environ 50%. En outre le procédé décrit dans ce document génère des quantités élevées de rejets organiques, ce qui n'est pas acceptable pour une mise en œuvre dans un procédé industriel à grande échelle. De plus, le produit obtenu a une pureté trop faible pour être utilisable dans les applications habituelles dans le caoutchouc.

Le document US2406960 cité ci-dessus décrit un procédé dont la source de zinc est soit du chlorure de zinc soit du sulfate de zinc. Ce procédé nécessite en outre l'emploi d'agents tampon, de type sels d'acide faible, tels que l'acétate de sodium, qui se retrouve intégralement dans les effluents aqueux. Aussi ce type de procédé n'est-il pas plus respectueux de l'environnement et ne peut donc être utilisé dans le cadre d'une production à grande échelle.

Le document EP1142869 décrit, quant à lui, un procédé de fabrication de dithiocarbamates de zinc, dont le ZBEC, à partir de sulfate de zinc, sans agent tampon organique susceptible de générer des effluents néfastes pour l'environnement et conduisant à une granulométrie médiane très élevée, de l'ordre de 100 µm, et donc avec une productivité élevée. Cependant, la qualité du produit obtenu par ce type de procédé n'est pas satisfaisante pour une application dans le caoutchouc, en raison notamment de la pureté insuffisante obtenue qui est généralement de l'ordre de seulement 80% à 85%, contrairement à ce qui est annoncé dans ce document.

Il reste donc un besoin pour un procédé permettant la fabrication à l'échelle industrielle de dithiocarbamates de zinc, qui s'affranchit des problèmes rencontrés dans les procédés connus de l'art antérieur, et en particulier avec une productivité maximale, des effluents respectueux de l'environnement et en particulier des rejets non chlorurés, des rendements maximaux pour éviter le rejets organiques, et tout particulièrement une qualité de produit conforme aux exigences de l'industrie du caoutchouc, à savoir tout particulièrement une granulométrie médiane en volume, régulière et fine et une pureté satisfaisante, celle-ci étant généralement caractérisée, dans le cas d'une application dans l'industrie du caoutchouc, par un point de fusion supérieur à 180°C environ.

Le problème technique à résoudre est donc de trouver un compromis entre un procédé à haute productivité, ne générant pas ou peu d'effluents néfastes pour l'environnement, et un procédé permettant l'obtention d'un produit de grande pureté et de granulométrie médiane adaptée à l'utilisation envisagée.

Un autre objectif encore est la mise à disposition d'un procédé de préparation de dithiocarbamates métalliques conduisant à un produit de grande pureté, typiquement une pureté supérieure ou égale à 96% en poids, ou tout au moins de pureté satisfaisante pour l'application principale envisagée comme accélérateur de vulcanisation, notamment pour les caoutchoucs.

Ainsi la présente invention a-t-elle pour objectif un procédé ne générant pas d'effluent chloruré, générant très peu d'effluents organiques et conduisant à un produit de granulométrie médiane en volume comprise entre 50 µm et 150 µm, typiquement entre 60 µm et 100 µm, afin de maintenir une bonne productivité, tout en conservant ses autres caractéristiques à des valeurs acceptables pour l'application, notamment comme accélérateur de vulcanisation, par exemple, dans le cas du ZBEC, une pureté supérieure à au moins 96% et un point de fusion supérieur à environ 180°C au moins (correspondant aux standards de l'industrie du caoutchouc).

Il doit être compris que ces deux caractéristiques, pureté et point de fusion, sont intimement liées et que généralement la mesure du point de fusion par une méthode standardisée telle que la DSC (*Differential Scanning Calorimetry)* est utilisée dans l'industrie du caoutchouc pour s'assurer d'une pureté suffisante du produit (cf. J. Grenet et B. Legendre, « Analyse calorimétrique différentielle à balayage (DSC) », Techniques de l'Ingénieur, Rapport P1205 du 10/12/2010).

Il a maintenant été découvert que les objectifs précités peuvent être atteints en totalité ou tout au moins en partie grâce à l'invention qui va maintenant être présentée. Le procédé selon la présente invention permet notamment la préparation d'un produit de granulométrie très élevée, typiquement supérieure à environ 50 µm à 60 µm, donc permettant une productivité élevée.

L'obtention d'un dithiocarbamate métallique présentant une très bonne apparence visuelle, et notamment d'un dithiocarbamate métallique de couleur blanche, représente encore un autre objectif de la présente invention. D'autres objectifs encore apparaîtront dans la description de l'invention qui suit.

Dans la présente invention, on entend de préférence par « dithiocarbamate métallique » un composé de formule générale (1) :

[(RR')N-C(=S)-S-]ₙMₘYₚ (1)

dans laquelle :
- R et R', identiques ou différents, sont choisis indépendamment l'un de l'autre parmi l'atome d'hydrogène et un radical hydrocarboné saturé ou totalement ou partiellement insaturé, linéaire, ramifié ou cyclique, contenant de 1 à 30 atomes de carbone, étant entendu que l'un au moins des radicaux R et R' est différent de l'atome d'hydrogène, et de préférence chacun des radicaux R et R' est différent de l'atome d'hydrogène,
   ou bien R et R' forment ensemble, et avec l'atome d'azote auquel ils sont reliés, un radical hétérocyclique à 5, 6, 7 ou 8 chaînons,
- M représente un atome métallique,
- Y est choisi parmi un atome d'oxygène, un atome de soufre, et un ligand provenant soit de la matière première soit du procédé de fabrication choisi, par exemple un groupe sulfate,
- n représente un entier compris entre 1 et 4, bornes incluses,
- m un entier compris entre 1 et 4, de préférence 1, 2 ou 3, de préférence encore 1 ou 2, bornes incluses, et
- p représente un entier compris entre 0 et 4, bornes incluses.

Il doit être compris que les composés de formule (1) ci-dessus sont des dithiocarbamates électroniquement neutres, c'est-à-dire que n et p varient en fonction du nombre m d'atomes métalliques présents dans la formule (1), ainsi que de la valence du métal ou des métaux présent(s) dans la formule (1).

Selon un mode de réalisation préféré, la présente invention concerne le procédé de préparation d'un dithiocarbamate métallique de formule générale (1a) :

[(RR')N-C(=S)-S-]nMm (1a),

cas particulier des composés de formule (1), dans laquelle p est égal à 0.

Parmi les radicaux hydrocarbonés R et R' saturés ou totalement ou partiellement insaturés, linéaires, ramifiés ou cycliques, contenant de 1 à 30 atomes de carbone, on peut citer à titre d'exemples non limitatifs le radicaux méthyle, éthyle, et les radicaux linéaires ou ramifiés propyles, butyles, pentyles, hexyles, heptyles, octyles, nonyles, décyles, undécyles, dodécyles, chaînes hydrocarbonées grasses saturées ou insaturées, phényle, benzyle, phénéthyle, naphtyle, et autres.

Des exemples de dithiocarbamates comprennent, de manière non limitative, les dibenzyldithiocarbamates (R = R' = benzyle), les penta-méthylène dithiocarbamates (R et R' formant ensemble le radical divalent -CH₂(CH₂)₃CH₂-), les hexa-méthylène dithiocarbamates (R et R' formant ensemble le radical divalent -CH₂(CH₂)₄CH₂-), les diamyldithiocarbamates, les di-(2-éthylhexyl)dithiocarbamates, les di-iso-nonyldithiocarbamates, les di-cocodithiocarbamates, les dioléyldithiocarbamates, les di-tallow-dithiocarbamates. Les dithiocarbamates préférés dans le cadre de la présente invention sont ceux pour lesquels R et R' représentent chacun un radical hydrocarboné tel que défini ci-dessus et de préférence encore ceux pour lesquels R et R' sont identiques.

L'atome métallique M peut être théoriquement choisi parmi tous les métaux du Tableau Périodique des Éléments, et avantageusement parmi les métaux des groupes 3 à 16 du Tableau Périodique des Éléments. Des exemples illustratifs mais non limitatifs de métal (M) du dithiocarbamate métallique préparé selon le procédé de la présente invention sont ceux pour lesquels ledit métal est choisi parmi le zinc, le nickel, le cuivre, le manganèse, le molybdène, le palladium, l'arsenic, le tellure, le chrome, le cadmium, le mercure, le plomb, le fer, le cobalt, l'antimoine, le bismuth et autres. Le procédé de la présente invention est tout particulièrement adapté à la préparation des dithiocarbamates de zinc.

À titre d'exemples non limitatifs :
- un dithiocarbamate de zinc peut être représenté par la formule (1_{Zn}) suivante : 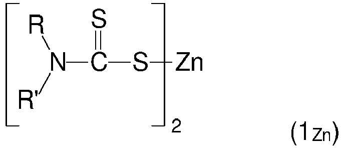 cas particulier du composé de formule (1) dans laquelle R et R' sont tels que définis précédemment et n représente 2, M représente le zinc, m représente 1 et p est égal à 0,
- un dithiocarbamate de molybdène peut être représenté par la formule (1 _{Mo}) suivante : 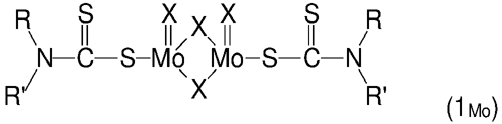 cas particulier du composé de formule (1) dans laquelle R et R' sont tels que définis précédemment et n représente 2, M représente le molybdène, m représente 2, X représente l'atome d'oxygène ou de soufre et p est égal à 4.

Il doit être entendu que les espèces Y et X définies dans les formules (1) et (1_{Mo}) respectivement peuvent être identiques ou différentes. En effet les dithiocarbamates définis dans la présente description, et typiquement le composé de formule (1_{Mo}), possèdent sont des mélanges complexes d'espèces Y (ou X) respectivement, identiques ou différentes.

D'autres dithiocarbamates qui peuvent être préparés selon le procédé de la présente invention sont, à tire d'exemples illustratifs et non limitatifs, le tris-(diéthyldithiocarbamate) d'arsenic (CAS n° 20393-93-5) et le tétrakis(diéthyldithio-carbamato)tellure (CAS n° 20941-65-5).

Ainsi, les dithiocarbamates qui peuvent être préparés selon le procédé de la présente invention peuvent être de tout type répondant à la formule (1) où le métal peut prendre tous les degrés de valence permis pour ledit métal. Par exemple, les dithiocarbamates de molybdène, dans lesquels le molybdène peut prendre tous les degrés de valence de 2 à 6, peuvent conserver un certain nombre d'atomes d'oxygène et/ou de soufre provenant des matières premières utilisées pour leur fabrication, telles que par exemple MoO₃ et/ou NaSH respectivement, matières premières qui sont par exemple décrites dans le document EP0727429.

La caractéristique du procédé selon l'invention pour préparer des dithiocarbamates métalliques est qu'il met en œuvre un complexe amine/sel métallique, où le sel métallique est un sel de métal, ledit métal étant de préférence choisi parmi le cuivre, le nickel, le zinc, le molybdène, le tellure, le cobalt, le manganèse, le mercure, l'antimoine et le bismuth, ce qui permet l'accès aux dithiocarbamates des métaux correspondants, et qui sont les dithiocarbamates métalliques les plus couramment utilisés, notamment dans l'industrie du caoutchouc et de la lubrification.

Ainsi, et selon un premier aspect, l'invention concerne un procédé de préparation d'un dithiocarbamate métallique, ledit procédé comprenant au moins les étapes réactionnelles suivantes, et de préférence consistant en les étapes réactionnelles suivantes :
Étape 1) formation d'un complexe amine/sel métallique par réaction entre un sel métallique et une amine, de préférence une amine secondaire, en milieu aqueux ou hydro-organique, de préférence en milieu aqueux ou hydro-alcoolique, à une température généralement comprise entre 0°C et 100°C, de préférence entre 10°C et 30°C ;
Étape 2) formation du dithiocarbamate métallique par ajout de sulfure de carbone en excès molaire compris entre 1,1 et 4 équivalents par rapport à l'amine dans le milieu réactionnel contenant le complexe amine/sel métallique formé à l'Étape 1, à une température comprise entre 0°C et 100°C, de préférence entre 10°C et 30°C ;
Étape 3) étape de finition de la réaction, sous agitation, pendant une durée comprise entre quelques minutes et quelques heures, voire quelques dizaines de minutes et quelques heures, à une température comprise entre 30°C et 100°C, de préférence entre 30°C et 40°C; et
Étape 4) filtration, lavage avec de l'eau à environ 70-80°C, séchage et récupération dudit dithiocarbamate métallique ; et
   dans lequel les Étapes 2 et 3 sont conduites en maintenant le pH du milieu réactionnel à une valeur comprise entre 5 et 8.

Selon un mode de réalisation préféré, l'invention concerne un procédé de préparation d'un dithiocarbamate métallique, dans lequel ledit dithiocarbamate métallique est un dithiocarbamate métallique de formule (1) telle que définie précédemment, ledit procédé comprenant au moins les étapes réactionnelles suivantes, et de préférence consistant en les étapes réactionnelles suivantes :
Étape 1) formation d'un complexe amine/sel métallique par réaction entre un sel métallique et une amine de formule RR'NH, où R et R' sont tels que définis précédemment, en milieu aqueux ou hydro-organique, de préférence en milieu aqueux ou hydro-alcoolique, à une température généralement comprise entre 0°C et 100°C, de préférence entre 10°C et 30°C ;
Étape 2) formation du dithiocarbamate métallique par ajout de sulfure de carbone en excès molaire compris entre 1,1 et 4 équivalents par rapport à l'amine dans le milieu réactionnel contenant le complexe amine/sel métallique formé à
l'Étape 1, à une température comprise entre 0°C et 100°C, de préférence entre 10°C et 30°C ;
Étape 3) étape de finition de la réaction, sous agitation, pendant une durée comprise entre quelques minutes, voire quelques dizaines de minutes à quelques heures, à une température comprise entre 30°C et 100°C, de préférence entre 30°C et 40°C; et
Étape 4) filtration, lavage avec de l'eau à environ 70-80°C, séchage et récupération du sel métallique de dithiocarbamate (ou dithiocarbamate métallique) ; et dans lequel les Étapes 2 et 3 sont conduites en maintenant le pH du milieu réactionnel à une valeur comprise entre 5 et 8.

Les Étapes 2 et 3 ci-dessus sont avantageusement et de préférence conduites en maintenant le pH du milieu réactionnel à une valeur comprise entre 5 et 8, de préférence entre 5,5 et 7,5 soit par l'addition, en une seule ou en plusieurs fois ou en continu, de préférence en continu, d'une solution d'un acide ou d'une base, ou bien par l'ajout d'un agent tampon. En outre, pour améliorer la précision du contrôle du pH, et notamment dans le cas de l'ajout, plus particulièrement en continu, d'un acide ou d'une base, on choisira de préférence une solution diluée de concentration inférieure ou égale à par exemple 50%, de préférence inférieure à 20% et idéalement inférieure à 10% en masse d'acide ou de base respectivement dans un solvant de dilution, qui peut être un solvant aqueux ou hydro-organique, de préférence un solvant aqueux ou hydro-alcoolique.

Il a été découvert, de façon surprenante, que l'excès de sulfure de carbone dans l'Étape 2 est un paramètre important, voire nécessaire à respecter pour obtenir un produit final présentant la qualité désirée.

De manière tout à fait avantageuse, il a été observé que d'une part l'excès de sulfure de carbone dans l'Étape 2, comme indiqué ci-dessus, mais aussi le contrôle du pH lors des Étapes 2 et 3 sont les paramètres à respecter pour obtenir un produit final présentant la qualité désirée.

Dans un mode de réalisation tout à fait préféré, les Étapes 1 à 3 décrites ci-dessus sont réalisées dans le même réacteur, sans isoler le complexe intermédiaire amine/sel métallique, autrement dit le procédé de l'invention peut être qualifié de réaction en un pot, ou « one-pot reaction », en langue anglaise.

La formation du complexe amine/sel métallique de l'Étape 1) est réalisée par mélange, avantageusement stœchiométrique (1 mole d'amine pour 0,5 mole de sel métallique), de l'amine ou d'un mélange amine/eau et d'une solution aqueuse de sel métallique. Lorsque l'amine n'est pas soluble en milieu aqueux, la formation du complexe amine/sel métallique de l'Étape 1) peut être réalisée en présence d'au moins un agent compatibilisant.

Dans ce mode de réalisation, ledit au moins un agent compatibilisant ajouté peut être de tout type connu de l'homme du métier et peut notamment être choisi, à titre d'exemples non limitatifs, parmi les solvants, les alcools, les émulsifiants, les agents tensio-actifs et autres, ainsi que les mélanges de deux ou plusieurs d'entre eux en toutes proportions.

En ce qui concerne les agents compatibilisants, on peut notamment se référer par exemple à ceux décrits dans « *Tensioactifs* », Techniques de l'Ingénieur, Rapport K342 (1995), en particulier les tensio-actifs non ioniques, et notamment les hétérocycles azotés N-alkylés, les alkyl- ou aryl-phénols polyéthoxylés, les alcools gras polyéthoxylés, les esters d'acides gras polyéthoxylés, les amines grasses polyéthoxylées, et autres..., ou encore les tensio-actifs anioniques, et notamment les alkyl- ou alkylaryl-sulfonates, les sulfosuccinates, les alkyl- ou nonylphényl-éther sulfates, etc..., mais aussi les tensio-actifs cationiques, tels que par exemple les sels d'ammonium quaternaires.

Comme indiqué précédemment, l'Étape 1 met en œuvre une amine et un sel métallique pour former un complexe. Les amines qui peuvent être utilisées sont les amines primaires ou secondaires, et avantageusement et de préférence les amines secondaires.

Selon un mode de réalisation de l'invention, l'amine est une amine de formule RR'NH, où R et R', identiques ou différents, sont choisis indépendamment l'un de l'autre parmi l'atome d'hydrogène et un radical hydrocarboné saturé ou totalement ou partiellement insaturé, linéaire, ramifié ou cyclique, contenant de 1 à 30 atomes de carbone, étant entendu que l'un au moins des radicaux R et R' est différent de l'atome d'hydrogène, et de préférence chacun des radicaux R et R' est différent de l'atome d'hydrogène,
ou bien R et R' forment ensemble, et avec l'atome d'azote auquel ils sont reliés, un radical hétérocyclique à 5, 6, 7 ou 8 chaînons.

Des exemples non limitatifs d'aminés qui peuvent être utilisées dans le cadre de la présente invention comprennent la diméthylamine, la diéthylamine, la dipropylamine, la dibutylamine, la pipéridine, la morpholine, la N-méthylpipérazine, la N-éthylpipérazine, la dibenzylamine, les diarylamines telles que la diphénylamine, la N-éthylaniline, la N-méthylaniline, la méthyl-*tert*-butylamine, l'éthyl*-tert-*butylamine, l'hexaméthylène-imine, la diamylamine, les dioctylamines telles que la bis (2-éthylhexyl)amine, les dinonylamines telles que la diisononylamine, la dicocoamine, la dioléylamine et la ditallowamine.

Parmi ces amines, on préfère particulièrement les amines non aromatiques dites « lourdes », c'est-à-dire ayant un point d'ébullition sous pression atmosphérique supérieur à environ 250 °C et de préférence supérieur à environ 280 °C élevé , telles que la dibenzylamine, les dioctylamines telles que la bis (2-éthylhexyl)amine, les dinonylamines telles que la di-*iso*-nonylamine, la dicocoamine, la di-oléylamine ou la ditallowamine, et parmi celles-ci, la dibenzylamine est tout particulièrement préférée.

Le sel métallique contenant le cation métallique Mⁿ⁺, où M et n sont tels que définis précédemment est apporté sous forme de sel métallique préparé à partir d'un métal M et d'au moins un acide fort, de préférence un acide minéral fort, par exemple et de manière non limitative choisi parmi l'acide sulfurique, l'acide chlorhydrique, l'acide bromhydrique et l'acide nitrique, de préférence l'acide sulfurique, pour des raisons évidentes de protection de l'environnement.

Les sels métalliques utilisés sont en règle générale et de préférence des sels de métaux lourds, tels que ceux habituellement utilisés en tant qu'agents de précipitation pour la préparation de sels métalliques stables d'acides dithiocarbamiques.

De tels sels de métaux lourds sont bien connus de l'homme du métier. Comme indiqué précédemment, des exemples non limitatifs de sels de métaux lourds qui peuvent être avantageusement utilisés dans le cadre de la présente invention sont les sels de zinc, nickel, cuivre, manganèse, molybdène, palladium, arsenic, tellure, chrome, cadmium, mercure, plomb, fer, cobalt, antimoine, bismuth et autres. Le procédé de la présente invention est tout particulièrement adapté pour la préparation de dithiocarbamates de zinc.

En règle générale, la concentration molaire en cation métallique Mⁿ⁺ optimale dépend de plusieurs facteurs, et notamment de la concentration finale désirée du sel métallique d'acide dithiocarbamique, de la température de la réaction ou de la productivité désirée du procédé. Par concentration molaire, on entend le nombre de moles par litre de solution.

À titre d'exemple non limitatif, dans le cas du cation Zn²⁺, celui-ci est apporté par le sulfate de zinc, éventuellement sous forme d'hydrate, avantageusement en solution dans l'eau, en concentration molaire comprise entre 0,1 moles par litre et 4 moles par litre de solution, et de préférence entre 0,4 moles par litre et 0,8 moles par litre de solution.

La préparation du complexe amine/sel métallique est avantageusement réalisée sous agitation. Le système d'agitation peut être de tout type connu de l'homme du métier, et par exemple et de manière non limitative, un agitateur à pales, de préférence un agitateur multi-pales, par exemple tripales. La vitesse d'agitation dépend de nombreux facteurs, dont par exemple la concentration et la température du milieu.

En règle générale, la vitesse d'agitation est comprise entre environ 100 tours/min et environ 800 tours/min, de préférence entre environ 300 tours/min et environ 700 tours/min, bornes incluses. Dans le cas d'un agitateur à pales, la vitesse d'agitation en bout de pale est généralement comprise entre environ 0,4 m.s⁻¹ et environ 3,5 m.s⁻¹, de préférence entre environ 1,2 m.s⁻¹ et environ 3 m.s⁻¹, bornes incluses.

Le procédé de l'invention peut être réalisé à toute température, dans les gammes indiquées précédemment. Pour des raisons évidentes de facilités de mise en œuvre, on préfère toutefois réaliser le procédé dans des gammes de températures où les différentes solutions et milieux réactionnels sont à l'état liquide. On peut en outre prévoir de chauffer légèrement les solutions afin de faciliter et/ou augmenter la solubilité des sels.

Le procédé selon l'invention est généralement réalisé à pression atmosphérique, mais on ne sortirait pas du cadre de l'invention si le procédé était opéré sous légère dépression ou sous légère pression. Le procédé de l'invention n'implique en effet pas de phase gazeuse, de sorte que la pression appliquée au milieu réactionnel n'a que peu d'effet, voire est sans effet.

Dans l'Étape 2, le disulfure de carbone (CS₂) est ajouté en excès par rapport à la stœchiométrie de la réaction dans le milieu réactionnel contenant le complexe obtenu à l'Étape 1, c'est-à-dire en excès molaire par rapport à l'amine. L'excès molaire de CS₂ est ainsi compris entre 1,1 et 4, de préférence entre 1,1 et 3, de préférence encore entre 1,1 et 2, de préférence encore entre 1,2 et 1,8 équivalents.

Par exemple, dans le cas de la préparation du dibenzyldithiocarbamate de zinc (ZBEC) l'excès molaire de sulfure de carbone est typiquement compris entre 1,2 et 1,6 équivalent, de préférence entre 1,3 et 1,6 équivalent par rapport à l'amine. Un excès molaire inférieur à 1,2 conduit à une pureté du ZBEC qui peut être inférieure à 90%, ce qui est en-dessous de la limite acceptable pour les applications classiques dans le domaine du caoutchouc. Dans le cas d'un excès molaire supérieur à 1,6 équivalent de CS₂, il n'y a pas d'amélioration substantielle des performances du procédé (conversion de la dibenzylamine, rendement et qualité du ZBEC). De fait, s'il est théoriquement possible d'opérer avec un excès molaire supérieur à 1,6, de tels excès ne seront pas mis en œuvre pour des raisons évidentes de coûts, mais aussi de volumes de CS₂ mis en œuvre.

Après addition du sulfure de carbone, sous agitation, le milieu réactionnel peut être avantageusement chauffé sous courant d'azote, toujours sous agitation, à une température pouvant aller jusqu'à 100°C, de préférence entre 30°C et 50°C, pendant 30 minutes à 200 minutes, de préférence entre 30 minutes et 60 minutes. Cette étape de chauffage permet d'éliminer le sulfure de carbone en excès, celui-ci pouvant être éventuellement et avantageusement recyclé pour des lots suivants de préparation de dithiocarbamates.

Comme indiqué précédemment, pendant les Étapes 2 et 3 du procédé, le pH du milieu réactionnel doit être maintenu en permanence à une valeur comprise entre 5 et 8, de préférence entre 5,5 et 7,5. Toutes les techniques connues de l'homme du métier peuvent être utilisées pour maintenir le pH du milieu réactionnel dans les valeurs indiquées ci-dessus, et par exemple, et à titre d'exemples non limitatifs, notamment dans le cas de la préparation du dibenzyldithiocarbamate de zinc (ZBEC), le pH du milieu réactionnel est maintenu à une valeur comprise entre 5 et 8 soit par l'addition en continu d'une solution diluée d'une base minérale, forte ou faible, de préférence une base minérale forte, préférentiellement l'hydroxyde de sodium ou de potassium, l'hydrogénocarbonate de sodium ou de potassium par exemple, soit par l'ajout d'un tampon non organique, tel qu'un tampon phosphate, dans le milieu réactionnel, celui-ci étant alors ajouté avant le début de la deuxième étape.

Après ces 3 étapes, le produit souhaité, précipité sous forme d'un solide en suspension, est ensuite filtré selon tout moyen classique connu de l'homme du métier, lavé à l'eau chaude, entre 50 et 90°C, de préférence entre 70 et 80°C, puis séché. La granulométrie médiane du produit ainsi obtenu est remarquablement élevée et est comprise entre environ 60 µm et quelques centaines de micromètres, selon le dithiocarbamate considéré, et par exemple la granulométrie médiane est comprise entre 60 µm et 150 µm.

Comme indiqué précédemment, et notamment dans le cas du dibenzyldithiocarbamate de zinc, le procédé de l'invention conduit à un produit de pureté tout à fait satisfaisante, contrairement à celui décrit dans le brevet EP1142869. Ce résultat tout à fait inattendu provient notamment du fait que :
- d'une part un excès de sulfure de carbone (CS₂) par rapport à l'amine est mis en œuvre, ce qui est compris comme permettant de réduire la présence d'amine résiduelle dans le produit final, et
- d'autre part le pH du milieu réactionnel est avantageusement contrôlé tout au long des Étapes 2 et 3, ce qui permet d'éviter d'atteindre des valeurs de pH très acides, qui sont très probablement préjudiciables au bon déroulement de la réaction et responsables de la présence d'impuretés dans le produit final ; il a en effet été observé que ceci peut être notamment le cas lorsque l'on neutralise le milieu réactionnel après les Étapes 2 et 3.

Ainsi, pour le procédé de la présente invention et par exemple dans le cas du dibenzyldithiocarbamate de zinc, la quantité de dibenzylamine dans le produit final, mesurée par RMN est inférieure à 1% en poids. À titre de comparaison, dans le procédé décrit dans le brevet EP1142869, qui utilise seulement 1,06 équivalents de CS₂ par rapport à l'amine engagée (soit 5% en poids par rapport au complexe amine/sulfate de zinc), et où le milieu est neutralisé après la formation du produit final, jusqu'à des valeurs de pH inférieures à 5, voire à 4, la quantité de dibenzylamine dans le produit final est comprise entre 10 et 15% en poids, ce qui n'est pas du tout acceptable pour les applications envisagées du dithiocarbamate de zinc.

Selon un mode de réalisation, le procédé de la présente invention permet d'obtenir un dithiocarbamate métallique qui satisfait au moins une, de préférence au moins deux et de préférence encore les trois caractéristiques suivantes :
- une granulométrie médiane des particules supérieure à 50 µm, de préférence supérieure à 60 µm d'une part, et inférieure à 200 µm, de préférence inférieure à 100 µm,
- une pureté supérieure ou égale à 99 %, mesurée par spectroscopie de Résonance Magnétique Nucléaire (RMN),
- un point de fusion supérieur à 180°C, mesuré par DSC (Differential Scanning Calorimetry).

Le produit obtenu se caractérise également par un produit doté d'une très bonne apparence visuelle (couleur blanche) et surtout une granulométrie médiane des particules supérieure à 50 µm, de préférence supérieure à 60 µm, ce qui permet d'améliorer très significativement la productivité du procédé par rapport aux procédés classiques de fabrication des dithiocarbamates métalliques.

Ainsi, dans le cas du dibenzyldithiocarbamate de zinc par exemple, les procédés classiques conduisent généralement à des produits significativement plus fins, dont la granulométrie médiane est typiquement inférieure ou égale à 5 µm, ce qui conduit à des temps de filtration qui peuvent aller jusqu'à plusieurs heures (voir exemple 2 du brevet EP1142869).

Comme déjà indiqué précédemment, le procédé de l'invention comprend 2 étapes réactionnelles, effectuées dans un seul et même réacteur, contrairement au procédé décrit dans EP1142869 qui comporte trois étapes réactionnelles, la troisième étape étant une étape de neutralisation du milieu réactionnel par une base.

La granulométrie des dithiocarbamates métalliques obtenus selon le procédé de la présente invention est mesurée à l'aide d'un granulomètre laser de type MALVERN Mastersizer 3000. Cet appareil permet d'analyser la granulométrie médiane en volume Dv50 ou x₅₀ exprimée en micromètres (µm) : cf. norme ISO 13320 : 2009(E).

Les cristaux obtenus à l'issu du procédé de la présente invention sont ensuite filtrés, selon toute méthode connue de l'homme du métier, et par exemple au moyen d'un filtre-presse, d'une essoreuse, ou d'une centrifugeuse. Les cristaux filtrés sont ensuite lavés à l'eau, de préférence à l'eau chaude (70-80°C), séchés selon toute méthode connue de l'homme du métier, avant d'être récupérés puis soumis, si besoin, à une opération de broyage afin de ramener la granulométrie médiane en volume des cristaux à des valeurs inférieures à 50 µm et dépendantes de l'application visée.

Par exemple, pour la vulcanisation des caoutchoucs classiques tels que par exemple les caoutchoucs naturels, Styrène-Butadiène, isoprène ou EPDM, cette granulométrie médiane pourra être comprise entre 5 µm et 50 µm environ ; pour la vulcanisation des latex, la granulométrie médiane pourra être comprise entre 1 µm et 10 µm.

Ce broyage peut être effectué selon tout moyen connu de l'homme du métier, et par exemple au moyen d'un ou plusieurs appareils choisis parmi les broyeurs à billes, les broyeurs à boulets et les broyeurs à jets d'air.

Les exemples suivants illustrent l'invention sans la limiter.

### Exemple 1 : (selon l'invention)

Dans un réacteur de 2,5 L en verre muni :
- d'un agitateur tripales (pales inclinées à 45°) de type Mixel de 77 mm de diamètre,
- d'une double enveloppe contenant un fluide caloporteur,
- d'une sonde de température,
- d'une sonde de pH,
- d'un réfrigérant alimenté par de l'eau à 5-10°C,
- d'une arrivée d'azote, et
- de 2 tubes d'introduction, l'un relié à une pompe péristaltique et à un flacon contenant le CS₂, l'autre relié à une ampoule de coulée,
on introduit 264 g (1,3 moles) de dibenzylamine, de pureté 97%, 0,7 g de 1-dodécyl-2-pyrrolidinone (émulsifiant non ionique) et 260 g d'eau.

Ce mélange est agité à environ 800 tr/mn et à température ambiante (18°C à 20°C). Le réacteur est maintenu sous léger balayage d'azote et la température du fluide caloporteur est fixée à température ambiante.

On ajoute ensuite en 1 heure, par l'intermédiaire de l'ampoule de coulée, une solution de sulfate de zinc heptahydraté (187,2 g soit 0,65 moles, dans 1040 g d'eau). Il se forme un précipité blanc correspondant au complexe dibenzylamine/sulfate de zinc. La température du milieu réactionnel est de l'ordre de 18°C à 21°C.

On introduit ensuite en 2 heures, 129 g (1,7 moles, soit 1,3 équivalent molaire par rapport à la dibenzylamine) de disulfure de carbone. Le pH est régulé en continu par ajout d'une solution aqueuse d'hydroxyde de sodium (NaOH) à 7,5% (préparée à partir de 52 g de NaOH en pastilles, soit 1,29 moles, dans 650 g d'eau), de manière à maintenir le pH entre 5,5 et 7,5 environ. La température du milieu réactionnel est de l'ordre de 18°C à 21°C.

À la fin de cet ajout, le mélange est chauffé sous agitation et sous azote à une température comprise entre 35°C et 40°C pendant 2 heures, en arrêtant l'alimentation de l'eau du réfrigérant et en maintenant le contrôle du pH en continu entre 5,5 et 7,5.

Le mélange est ensuite filtré, à une température comprise entre 35°C et 40°C, sur un filtre statique de laboratoire de type Nutsche (cf. par exemple : G. Mériguet, Techniques de l'ingénieur, « Filtration-Technologie », rapport J3510 (10 septembre 1997), page 27) en inox et équipé d'une toile filtrante en textile sous environ 1 bar (100 kPa) de pression. Après lavage du gâteau de filtration sur ce filtre par de l'eau à une température d'environ 70°C à 80°C, on obtient 587 g d'une poudre blanche humide qui est séchée pendant environ 12 heures dans une étuve portée à une température comprise entre 50°C et 60°C, sous vide (environ 10 mbar à 20 mbar, soit environ 1 kPa à 2 kPa).

On récupère 380 g d'une poudre blanche et sèche dont le point de fusion est de 184°C ; l'analyse par spectroscopie RMN indique une pureté de 99% environ et la présence d'environ 0,9% en poids de dibenzylamine. Le rendement massique en produit brut est de 96% environ. La granulométrie médiane du produit en volume Dv50 (taille pour laquelle 50% de l'échantillon en volume se trouve en dessous de cette dimension) est de 65 µm environ.

### Exemple 2 : (comparatif)

### Préparation du dibenzyldithiocarbamate de zinc dans les mêmes conditions que l'exemple 1 du document EP1142869

Dans le même réacteur que l'exemple ci-dessus, on introduit 256,1 g (1,26 moles) de dibenzylamine de pureté 97%, 0,7 g de 1-dodécyl-2-pyrrolidinone (émulsifiant non ionique) et 260 g d'eau. Ce mélange est agité à environ 800 tr.mn⁻¹ et à température ambiante (20°C à 21°C). Le réacteur est maintenu sous un léger balayage d'azote et le fluide caloporteur est à température ambiante.

On ajoute ensuite, en 1 heure et 20 minutes, par l'intermédiaire de l'ampoule de coulée, une solution de sulfate de zinc heptahydraté (187,2 g soit 0,65 moles, dans 1040 g d'eau). Il se forme un précipité blanc. La température du milieu réactionnel est de l'ordre de 22°C à 23°C.

On introduit ensuite, en 1 heure et 30 minutes, 102 g (1,34 moles, soit 1,06 équivalent molaire par rapport à la dibenzylamine) de disulfure de carbone. Le pH décroît régulièrement jusqu'à environ 4,7. La température du milieu réactionnel est de l'ordre de 23°C à 25°C.

À la fin de cet ajout, le mélange est chauffé sous agitation et sous azote à environ 35°C pendant 45 minutes ; ensuite, 702 g d'une solution aqueuse d'hydroxyde de sodium à 7,4% massique (préparée à partir de 52 g de soude en pastilles et 650 g d'eau) sont ajoutés en 45 minutes et la température monte à environ 35°C à 40°C. Après 1 heure d'agitation à environ 40°C en arrêtant l'alimentation de l'eau du réfrigérant, le mélange est filtré sur un filtre de laboratoire de type Nutsche comme indiqué précédemment en inox et équipé d'une toile filtrante en textile sous environ 1 bar (100 kPa) de pression.

Après lavage du gâteau de filtration sur ce filtre par de l'eau à 70°C à 80°C, on obtient 523 g d'une poudre blanche humide qui est ensuite séchée pendant environ 12 heures dans une étuve à 50°C à 60°C, sous vide (environ 10 mbar à 20mbar, soit environ 1 kPa à 2 kPa).

On récupère 368 g d'une poudre blanche et sèche dont le point de fusion est de 167°C ; l'analyse par spectroscopie RMN indique une pureté de 85% environ et la présence d'environ 15% de dibenzylamine. Le rendement massique en ZBEC est donc de 81% environ. La granulométrie médiane du produit en volume, Dv50 (taille pour laquelle 50% de l'échantillon en volume se trouve en dessous de cette dimension) est de 65 µm environ.

### Exemple 3 : (comparatif)

### Fabrication du dibenzyldithiocarbamate de zinc dans les mêmes conditions que celles décrites dans le document CN102276509 :

Dans le même réacteur que l'exemple 1 ci-dessus, on introduit 1898 g d'eau, 48,1 g (0,59 moles) d'oxyde de zinc (Silox 2C de la société SILAR), 240,2 g (1,22 moles) de dibenzylamine de pureté 97% et 0,05 g d'un catalyseur constitué d'un mélange équimolaire d'acide stéarique, de stéarate de sodium et de stéarate de triglycéryle. Ce mélange est agité à environ 500 tr.mn⁻¹ et chauffé à environ 40°C sous un léger balayage d'azote.

On ajoute ensuite, en 2 heures, 91,3 g (1,2 moles) de disulfure de carbone (CS₂), en maintenant la température à 40°C. Le pH décroit à environ 2,5. Le milieu réactionnel est alors chauffé à 80°C pendant 1 heure en arrêtant l'alimentation de l'eau du réfrigérant pour éliminer le CS₂ non réagi.

On refroidit ensuite le milieu réactionnel à environ 35 °C et le mélange est filtré sur un filtre de laboratoire de type Nutsche comme décrit précédemment en inox et équipé d'une toile filtrante en textile sous environ 1 bar (100 kPa) de pression.

Après lavage du gâteau de filtration sur ce filtre par de l'eau à environ 20°C à 25°C, on obtient, après séchage dans une étuve ventilée à 65°C, 189 g d'une poudre très hétérogène comprenant quelques grains de taille millimétriques dont le point de fusion est de 167°C, caractéristique d'un produit de pureté inférieure à 90%.

Le rendement massique en produit brut est d'environ 51 % seulement. La granulométrie médiane du produit en volume Dv5O (taille pour laquelle 50% de l'échantillon se trouve en dessous de cette dimension) est de 65 µm environ.

## Revendications

1. Procédé de préparation d'un dithiocarbamate métallique, ledit procédé comprenant au moins les étapes réactionnelles suivantes :
Étape 1) formation d'un complexe amine/sel métallique par réaction entre un sel métallique et une amine, de préférence une amine secondaire, en milieu aqueux ou hydro-organique, de préférence en milieu aqueux ou hydro-alcoolique, à une température généralement comprise entre 0°C et 100°C, de préférence entre 10°C et 30°C ;
Étape 2) formation du dithiocarbamate métallique par ajout de sulfure de carbone en excès molaire compris entre 1,1 et 4 équivalents par rapport à l'amine,
dans le milieu réactionnel contenant le complexe amine/sel métallique formé à l'Étape 1, à une température comprise entre 0°C et 100°C, de préférence entre 10°C et 30°C ;
Étape 3) étape de finition de la réaction, sous agitation, pendant une durée comprise entre quelques minutes et quelques heures, voire quelques dizaines de minutes et quelques heures, à une température comprise entre 30°C et 100°C, de préférence entre 30°C et 40°C ;
Étape 4) filtration, lavage avec de l'eau à environ 70-80°C, séchage et récupération dudit sel métallique de dithiocarbamate ; et
dans lequel les Étapes 2 et 3 sont conduites en maintenant le pH du milieu réactionnel à une valeur comprise entre 5 et 8.

2. Procédé de préparation selon la revendication 1, dans lequel ledit dithiocarbamate métallique est représenté par la formule générale (1) :
[(RR')N-C(=S)-S-]nMmYp (1)
dans laquelle :
- R et R', identiques ou différents, sont choisis indépendamment l'un de l'autre parmi l'atome d'hydrogène et un radical hydrocarboné saturé ou totalement ou partiellement insaturé, linéaire, ramifié ou cyclique, contenant de 1 à 30 atomes de carbone, étant entendu que l'un au moins des radicaux R et R' est différent de l'atome d'hydrogène, ou bien R et R' forment ensemble, et avec l'atome d'azote auquel ils sont reliés, un radical hétérocyclique à 5, 6, 7 ou 8 chaînons,
- M représente un atome métallique,
- Y est choisi parmi un atome d'oxygène, un atome de soufre, et un ligand provenant soit de la matière première soit du procédé de fabrication choisi, par exemple un groupe sulfate,
- n représente un entier compris entre 1 et 4, bornes incluses,
- m un entier compris entre 1 et 4, de préférence 1, 2 ou 3, de préférence encore 1 ou 2, bornes incluses, et
- p représente un entier compris entre 0 et 4, bornes incluses,

3. Procédé selon la revendication 1, dans lequel l'amine est une amine de formule RR'NH, où R et R', identiques ou différents, sont choisis indépendamment l'un de l'autre parmi l'atome d'hydrogène et un radical hydrocarboné saturé ou totalement ou partiellement insaturé, linéaire, ramifié ou cyclique, contenant de 1 à 30 atomes de carbone, étant entendu que l'un au moins des radicaux R et R' est différent de l'atome d'hydrogène, et de préférence chacun des radicaux R et R' est différent de l'atome d'hydrogène, ou bien R et R' forment ensemble, et avec l'atome d'azote auquel ils sont reliés, un radical hétérocyclique à 5, 6, 7 ou 8 chaînons.

4. Procédé selon la revendication 1, dans lequel le dithiocarbamate métallique est un dithiocarbamate de formule générale (1a) :
[(RR')N-C(=S)-S-]nMm (1a),
dans laquelle :
- R et R', identiques ou différents, sont choisis indépendamment l'un de l'autre parmi l'atome d'hydrogène et un radical hydrocarboné saturé ou totalement ou partiellement insaturé, linéaire, ramifié ou cyclique, contenant de 1 à 30 atomes de carbone, étant entendu que l'un au moins des radicaux R et R' est différent de l'atome d'hydrogène, ou bien R et R' forment ensemble, et avec l'atome d'azote auquel ils sont reliés, un radical hétérocyclique à 5, 6, 7 ou 8 chaînons,
- M représente un atome métallique,
- n représente un entier compris entre 1 et 4, bornes incluses, et
- m un entier compris entre 1 et 4, de préférence 1, 2 ou 3, de préférence encore 1 ou 2, bornes incluses.

5. Procédé selon la revendication 1, dans lequel le métal du dithiocarbamate métallique est choisi parmi le zinc, le nickel, le cuivre, le manganèse, le molybdène, le palladium, l'arsenic, le tellure, le chrome, le cadmium, le mercure, le plomb, le fer, le cobalt, l'antimoine, le bismuth et autres.

6. Procédé selon la revendication 1, dans lequel le dithiocarbamate métallique est un dithiocarbamate de zinc représenté par la formule (1_{Zn}) : 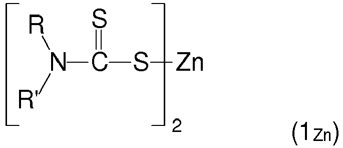 dans laquelle R et R', identiques ou différents, sont choisis indépendamment l'un de l'autre parmi l'atome d'hydrogène et un radical hydrocarboné saturé ou totalement ou partiellement insaturé, linéaire, ramifié ou cyclique, contenant de 1 à 30 atomes de carbone, étant entendu que l'un au moins des radicaux R et R' est différent de l'atome d'hydrogène, ou bien R et R' forment ensemble, et avec l'atome d'azote auquel ils sont reliés, un radical hétérocyclique à 5, 6, 7 ou 8 chaînons.

7. Procédé selon la revendication 1, dans lequel ledit dithiocarbamate métallique est un dithiocarbamate de molybdène représenté par la formule (1_{Mo}) : 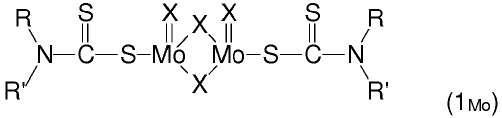 dans laquelle :
- R et R', identiques ou différents, sont choisis indépendamment l'un de l'autre parmi l'atome d'hydrogène et un radical hydrocarboné saturé ou totalement ou partiellement insaturé, linéaire, ramifié ou cyclique, contenant de 1 à 30 atomes de carbone, étant entendu que l'un au moins des radicaux R et R' est différent de l'atome d'hydrogène, ou bien R et R' forment ensemble, et avec l'atome d'azote auquel ils sont reliés, un radical hétérocyclique à 5, 6, 7 ou 8 chaînons, et
- X représente l'atome d'oxygène ou de soufre, chacune des espèces X présentes pouvant être identiques ou différentes.

8. Procédé selon la revendication 1, dans lequel ledit dithiocarbamate métallique est le tris(diéthyldithiocarbamate) d'arsenic.

9. Procédé selon la revendication 1, dans lequel ledit dithiocarbamate métallique est le tétrakis(diéthyldithiocarbamato)tellure.

10. Procédé selon la revendication 1, dans lequel l'excès molaire de disulfure de carbone ajouté dans l'Étape 2, par rapport à l'amine est compris entre 1,1 et 4, de préférence entre 1,1 et 3, de préférence encore entre 1,1 et 2, de préférence encore entre 1,2 et 1,8 équivalents.

11. Procédé selon la revendication 1, dans lequel les Étapes 2 et 3 sont conduites en maintenant le pH du milieu réactionnel à une valeur comprise entre 5,5 et 7,5.

12. Procédé selon la revendication 1, dans lequel la formation du complexe amine/sel métallique de l'Étape 1) est réalisée en présence d'au moins un agent compatibilisant, de préférence choisi parmi les hétérocycles azotés N-alkylés, les alkyl- ou aryl-phénols polyéthoxylés, les alcools gras polyéthoxylés, les esters d'acides gras polyéthoxylés, les amines grasses polyéthoxylées, les tensio-actifs anioniques, et notamment les alkyl- ou alkylaryl-sulfonates, les sulfosuccinates, les alkyl- ou nonylphényl-éther sulfates, et les tensio-actifs cationiques, notamment les sels d'ammonium quaternaires.

13. Procédé selon la revendication 1, dans lequel l'amine est choisie parmi la diméthylamine, la diéthylamine, la dipropylamine, la dibutylamine, la pipéridine, la morpholine, la N-méthylpipérazine, la N-éthylpipérazine, la dibenzylamine, les diarylamines telles que la diphénylamine, la N-éthylaniline, la N-méthylaniline, la méthyl-*tert*-butylamine, l'éthyl-*tert*-butylamine, l'hexaméthylène-imine, la diamylamine, les dioctylamines telles que la bis (2-éthylhexyl)amine, les dinonylamines telles que la diisononylamine, la dicocoamine, la dioléylamine et la ditallowamine.

## Patentansprüche

1. Verfahren zur Herstellung eines Metall-Dithiocarbamats, wobei das Verfahren mindestens die folgenden Reaktionsschritte umfasst:
Schritt 1) Herstellung eines Amin/Metallsalz-Komplexes durch Reaktion zwischen einem Metallsalz und einem Amin, vorzugsweise einem sekundären Amin, in wässrigem oder wässrig-organischem Medium, vorzugsweise in wässrigem oder wässrig-alkoholischem Medium, bei einer Temperatur im Allgemeinen zwischen 0 °C und 100 °C, vorzugsweise zwischen 10 °C und 30 °C;
Schritt 2) Herstellung des Metall-Dithiocarbamats durch Zugabe von Kohlenstoffdisulfid in einem molaren Überschuss zwischen 1,1 und 4 Äquivalenten in Bezug auf das Amin in das Reaktionsmedium, das den in Schritt 1 hergestellten Amin/Metallsalz-Komplex enthält, bei einer Temperatur zwischen 0 °C und 100 °C, vorzugsweise zwischen 10 °C und 30 °C;
Schritt 3) Abschlussschritt der Reaktion unter Rühren während einer Dauer zwischen einigen Minuten und einigen Stunden oder sogar einigen zehn Minuten und einigen Stunden bei einer Temperatur zwischen 30 °C und 100 °C, vorzugsweise zwischen 30 °C und 40 °C;
Schritt 4) Filtration, Waschen mit Wasser bei etwa 70-80 °C, Trocknen und Gewinnen des Metallsalz-Dithiocarbamats, und
wobei die Schritte 2 und 3 unter Aufrechterhalten des pH-Werts des Reaktionsmediums bei einem Wert zwischen 5 und 8 durchgeführt werden.

2. Verfahren zur Herstellung nach Anspruch 1, wobei das Metall-Dithiocarbamat durch die allgemeine Formel (1) dargestellt wird:
[(RR')N-C(=S)-S-]nMmYp (1),
worin:
- R und R', die gleich oder verschieden sind, unabhängig voneinander aus einem Wasserstoffatom und einem gesättigten oder ganz oder teilweise ungesättigten, geraden, verzweigten oder zyklischen Kohlenwasserstoffrest mit 1 bis 30 Kohlenstoffatomen ausgewählt sind, mit der Maßgabe, dass mindestens einer der Reste R und R' kein Wasserstoffatom ist, oder R und R' zusammen und mit dem Stickstoffatom, an das sie gebunden sind, einen heterozyklischen Rest mit 5, 6, 7 oder 8 Ringgliedern bilden,
- M für ein Metallatom steht,
- Y aus einem Sauerstoffatom, einem Schwefelatom und einem Liganden, der entweder aus dem Ausgangsmaterial oder dem gewählten Herstellungsverfahren herrührt, zum Beispiel einer Sulfatgruppe, ausgewählt ist,
- n eine ganze Zahl zwischen 1 und 4 einschließlich der Grenzen darstellt,
- m eine ganze Zahl zwischen 1 und 4, vorzugsweise 1, 2 oder 3, noch stärker bevorzugt 1 oder 2 einschließlich der Grenzen und
- p eine ganze Zahl zwischen 0 und 4 einschließlich der Grenzen darstellt.

3. Verfahren nach Anspruch 1, wobei es sich bei dem Amin um ein Amin der Formel RR'NH handelt, worin R und R', die gleich oder verschieden sind, unabhängig voneinander aus einem Wasserstoffatom und einem gesättigten oder ganz oder teilweise ungesättigten, geraden, verzweigten oder zyklischen Kohlenwasserstoffrest mit 1 bis 30 Kohlenstoffatomen ausgewählt sind, mit der Maßgabe, dass mindestens einer der Reste R und R' kein Wasserstoffatom ist und vorzugsweise jeder der Reste R und R' kein Wasserstoffatom ist, oder R und R' zusammen und mit dem Stickstoffatom, an das sie gebunden sind, einen heterozyklischen Rest mit 5, 6, 7 oder 8 Ringgliedern bilden.

4. Verfahren nach Anspruch 1, wobei das Metall-Dithiocarbamat ein Dithiocarbamat der allgemeinen Formel (1a) ist:
**[(RH')N-C(=S)-S-]nMm** **(1a),**
worin:
- R und R', die gleich oder verschieden sind, unabhängig voneinander aus einem Wasserstoffatom und einem gesättigten oder ganz oder teilweise ungesättigten, geraden, verzweigten oder zyklischen Kohlenwasserstoffrest mit 1 bis 30 Kohlenstoffatomen ausgewählt sind, mit der Maßgabe, dass mindestens einer der Reste R und R' kein Wasserstoffatom ist, oder R und R' zusammen und mit dem Stickstoffatom, an das sie gebunden sind, einen heterozyklischen Rest mit 5, 6, 7 oder 8 Ringgliedern bilden,
- M für ein Metallatom steht,
- n eine ganze Zahl zwischen 1 und 4 einschließlich der Grenzen und
- m eine ganze Zahl zwischen 1 und 4, vorzugsweise 1, 2 oder 3, noch stärker bevorzugt 1 oder 2 einschließlich der Grenzen darstellt.

5. Verfahren nach Anspruch 1, wobei das Metall des Metall-Dithiocarbamats aus Zink, Nickel, Kupfer, Mangan, Molybdän, Palladium, Arsen, Tellur, Chrom, Cadmium, Quecksilber, Blei, Eisen, Kobalt, Antimon, Wismut und anderen ausgewählt ist.

6. Verfahren nach Anspruch 1, wobei das Metall-Dithiocarbamat ein Zink-Dithiocarbamat der Formel (1_{Zn}) ist: 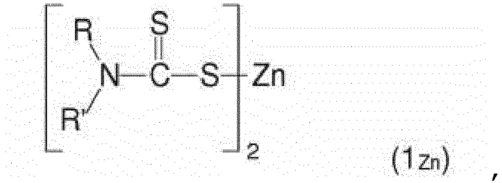 worin R und R', die gleich oder verschieden sind, unabhängig voneinander aus einem Wasserstoffatom und einem gesättigten oder ganz oder teilweise ungesättigten, geraden, verzweigten oder zyklischen Kohlenwasserstoffrest mit 1 bis 30 Kohlenstoffatomen ausgewählt sind, mit der Maßgabe, dass mindestens einer der Reste R und R' kein Wasserstoffatom ist, oder R und R' zusammen und mit dem Stickstoffatom, an das sie gebunden sind, einen heterozyklischen Rest mit 5, 6, 7 oder 8 Ringgliedern bilden.

7. Verfahren nach Anspruch 1, wobei das Metall-Dithiocarbamat ein Molybdän-Dithiocarbamat ist, das durch die Formel (1_{Mo}) dargestellt wird: 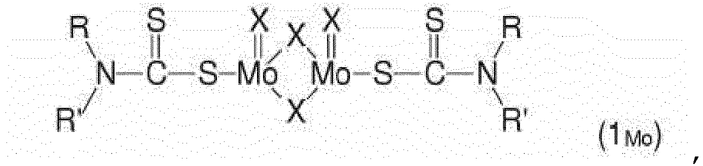 worin:
- R und R', die gleich oder verschieden sind, unabhängig voneinander aus einem Wasserstoffatom und einem gesättigten oder ganz oder teilweise ungesättigten, geraden, verzweigten oder zyklischen Kohlenwasserstoffrest mit 1 bis 30 Kohlenstoffatomen ausgewählt sind, mit der Maßgabe, dass mindestens einer der Reste R und R' kein Wasserstoffatom ist, oder R und R' zusammen und mit dem Stickstoffatom, an das sie gebunden sind, einen heterozyklischen Rest mit 5, 6, 7 oder 8 Ringgliedern bilden und
- X ein Sauerstoff- oder Schwefelatom darstellt, wobei die vorhandenen Spezies X jeweils gleich oder verschieden sein können.

8. Verfahren nach Anspruch 1, wobei es sich bei dem Metall-Dithiocarbamat um Arsentris(diethyldithiocarbamat) handelt.

9. Verfahren nach Anspruch 1, wobei es sich bei dem Metall-Dithiocarbamat um Tetrakis(diethyldithiocarbamato)tellur handelt.

10. Verfahren nach Anspruch 1, wobei der in Schritt 2 zugegebene molare Überschuss an Kohlenstoffdisulfid in Bezug auf das Amin zwischen 1,1 und 4, vorzugsweise zwischen 1,1 und 3, noch stärker bevorzugt zwischen 1,1 und 2, noch stärker bevorzugt zwischen 1,2 und 1,8 Äquivalenten beträgt.

11. Verfahren nach Anspruch 1, wobei die Schritte 2 und 3 unter Aufrechterhaltung des pH-Werts des Reaktionsmediums bei einem Wert zwischen 5,5 und 7,5 durchgeführt werden.

12. Verfahren nach Anspruch 1, wobei die Herstellung des Amin/Metallsalz-Komplexes von Schritt 1) in Gegenwart mindestens eines Kompatibilisierungsmittels durchgeführt wird, das vorzugsweise aus N-alkylierten stickstoffhaltigen Heterozyklen, polyethoxylierten Alkyl- oder Arylphenolen, polyethoxylierten Fettalkoholen, polyethoxylierten Fettsäureestern, polyethoxylierten Fettaminen, anionischen Tensiden und insbesondere Alkyl- oder Alkylarylsulfonaten, Sulfosuccinaten, Alkyl- oder Nonylphenylethersulfaten und kationischen Tensiden, insbesondere quaternären Ammoniumsalzen ausgewählt ist.

13. Verfahren nach Anspruch 1, wobei das Amin aus Dimethylamin, Diethylamin, Dipropylamin, Dibutylamin, Piperidin, Morpholin, N-Methylpiperazin, N-Ethylpiperazin, Dibenzylamin, Diarylaminen, wie Diphenylamin, N-Ethylanilin, N-Methylanilin, Methyl-tert-butylamin, Ethyl-tert-butylamin, Hexamethylenimin, Diamylamin, Dioctylaminen, wie Bis(2-ethylhexyl)amin, Dinonylaminen, wie Diisononylamin, Dicocoamin, Dioleylamin und Ditalgamin ausgewählt ist.

## Claims

1. Process for preparing a metal dithiocarbamate, said process comprising at least the following reaction steps:
Step 1) formation of an amine/metal salt complex by reaction between a metal salt and an amine, preferably a secondary amine, in aqueous or aqueous-organic medium, preferably in aqueous or aqueous-alcoholic medium, at a temperature generally between 0°C and 100°C, preferably between 10°C and 30°C;
Step 2) formation of the metal dithiocarbamate by adding carbon sulfide in a molar excess of between 1.1 and 4 equivalents relative to the amine, in the reaction medium containing the amine/metal salt complex formed in Step 1, at a temperature of between 0°C and 100°C, preferably between 10°C and 30°C;
Step 3) step of finishing the reaction, with stirring, for a time of between a few minutes and a few hours, or even several tens of minutes and several hours, at a temperature of between 30°C and 100°C, preferably between 30°C and 40°C;
Step 4) filtration, washing with water at about 70-80°C, drying and recovery of said dithiocarbamate metal salt; and
in which Steps 2 and 3 are performed while maintaining the pH of the reaction medium at a value of between 5 and 8.

2. Preparation process according to Claim 1, in which said metal dithiocarbamate is represented by the general formula (1):
[(RR')N-C(=S)-S-]ₙMₘYₚ (1)
in which:
- R and R', which may be identical or different, are chosen, independently of each other, from a hydrogen atom and a saturated or totally or partially unsaturated, linear, branched or cyclic hydrocarbon-based radical, containing from 1 to 30 carbon atoms, it being understood that at least one of the radicals R and R' is other than a hydrogen atom, or alternatively R and R' together form, with the nitrogen atom to which they are attached, a 5-, 6-, 7- or 8-membered heterocyclic radical,
- M represents a metal atom,
- Y is chosen from an oxygen atom, a sulfur atom and a ligand originating either from the starting material or from the manufacturing process, chosen, for example, from a sulfate group,
- n represents an integer between 1 and 4, limits inclusive,
- m is an integer between 1 and 4, preferably 1, 2 or 3, more preferably 1 or 2, limits inclusive, and
- p represents an integer between 0 and 4, limits inclusive.

3. Process according to Claim 1, in which the amine is an amine of formula RR'NH, in which R and R', which may be identical or different, are chosen, independently of each other, from a hydrogen atom and a saturated or totally or partially unsaturated, linear, branched or cyclic hydrocarbon-based radical, containing from 1 to 30 carbon atoms, it being understood that at least one of the radicals R and R' is other than a hydrogen atom, and preferably each of the radicals R and R' is other than a hydrogen atom, or alternatively R and R' together form, with the nitrogen atom to which they are attached, a 5-, 6-, 7- or 8-membered heterocyclic radical.

4. Process according to Claim 1, in which the metal dithiocarbamate is a dithiocarbamate of general formula (1a) :
[(RR')N-C(=S)-S-]ₙMₘ (1a)
in which:
- R and R', which may be identical or different, are chosen, independently of each other, from a hydrogen atom and a saturated or totally or partially unsaturated, linear, branched or cyclic hydrocarbon-based radical, containing from 1 to 30 carbon atoms, it being understood that at least one of the radicals R and R' is other than a hydrogen atom, or alternatively R and R' together form, with the nitrogen atom to which they are attached, a 5-, 6-, 7- or 8-membered heterocyclic radical,
- M represents a metal atom,
- n represents an integer between 1 and 4, limits inclusive, and
- m is an integer between 1 and 4, preferably 1, 2 or 3, more preferably 1 or 2, limits inclusive.

5. Process according to Claim 1, in which the metal of the metal dithiocarbamate is chosen from zinc, nickel, copper, manganese, molybdenum, palladium, arsenic, tellurium, chromium, cadmium, mercury, lead, iron, cobalt, antimony, bismuth and the like.

6. Process according to Claim 1, in which the metal dithiocarbamate is a zinc dithiocarbamate represented by formula (1zn) : 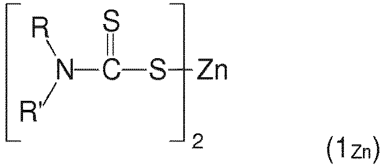 in which R and R', which may be identical or different, are chosen, independently of each other, from a hydrogen atom and a saturated or totally or partially unsaturated, linear, branched or cyclic hydrocarbon-based radical, containing from 1 to 30 carbon atoms, it being understood that at least one of the radicals R and R' is other than a hydrogen atom, or alternatively R and R' together form, with the nitrogen atom to which they are attached, a 5-, 6-, 7- or 8-membered heterocyclic radical.

7. Process according to Claim 1, in which said metal dithiocarbamate is a molybdenum dithiocarbamate represented by formula (1Mo): 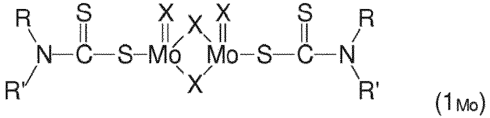 in which:
- R and R', which may be identical or different, are chosen, independently of each other, from a hydrogen atom and a saturated or totally or partially unsaturated, linear, branched or cyclic hydrocarbon-based radical, containing from 1 to 30 carbon atoms, it being understood that at least one of the radicals R and R' is other than a hydrogen atom, or alternatively R and R' together form, with the nitrogen atom to which they are attached, a 5-, 6-, 7- or 8-membered heterocyclic radical, and
- X represents an oxygen or sulfur atom, each of the species X present possibly being identical or different.

8. Process according to Claim 1, in which said metal dithiocarbamate is arsenic tris(diethyldithiocarbamate).

9. Process according to Claim 1, in which said metal dithiocarbamate is tetrakis(diethyldithiocarbamato)-tellurium.

10. Process according to Claim 1, in which the molar excess of carbon disulfide added in Step 2, relative to the amine, is between 1.1 and 4, preferably between 1.1 and 3, more preferably between 1.1 and 2, more preferably between 1.2 and 1.8 equivalents.

11. Process according to Claim 1, in which Steps 2 and 3 are performed while maintaining the pH of the reaction medium at a value of between 5.5 and 7.5.

12. Process according to Claim 1, in which the formation of the amine/metal salt complex of Step 1) is performed in the presence of at least one compatibilizer, preferably chosen from N-alkyl nitrogenous heterocycles, polyethoxylated alkylphenols or arylphenols, polyethoxylated fatty alcohols, polyethoxylated fatty acid esters, polyethoxylated fatty amines, anionic surfactants, and notably alkylsulfonates or alkylarylsulfonates, sulfosuccinates, alkyl or nonylphenyl ether sulfates, and cationic surfactants, notably quaternary ammonium salts.

13. Process according to Claim 1, in which the amine is chosen from dimethylamine, diethylamine, dipropylamine, dibutylamine, piperidine, morpholine, N-methylpiperazine, N-ethylpiperazine, dibenzylamine, diarylamines such as diphenylamine, N-ethylaniline, N-methylaniline, methyl-tert-butylamine, ethyl-tert-butylamine, hexamethyleneimine, diamylamine, dioctylamines such as bis(2-ethylhexyl)amine, dinonylamines such as diisononylamine, dicocoylamine, dioleylamine and ditallowamine.
